# EUROPEAN PATENT APPLICATION

(11) **EP 4 789 604 A1**
(43) Date of publication of application: **12.08.2026**
(21) Application number: 26156060.1
(22) Date of filing: 03.02.2026
(51) Int. Cl.: A61B 5/00, A61B 5/0205, G01S 5/02, G01S 5/10, G01S 11/06, G01S 13/74, G01S 13/82, G16H 40/67, H04B 1/69, H04W 4/02, H04W 4/80, H04W 48/20, H04W 64/00, H04W 76/10, H04W 76/14

(54) **SYSTEMS, DEVICES, AND METHODS FOR ESTABLISHING WIRELESS COMMUNICATION LINKS**

(30) Priority: 05.02.2025 US 202563754402 P
(71) Applicant: STRYKER CORPORATION, Portage, MI 49002-9711 (US)
(72) Inventor: ANDRÉ, Marc, Portage, 49002 (US); FEINGOLD, Benjamin Hyman, Portage, 49002 (US)
(74) Representative: V.O.

(57) **Abstract**

A method for establishing wireless communication between devices in a medical facility includes, at a first device located in a medical room of the medical facility, receiving at least one wireless signal from a second device; determining, based on the at least one wireless signal received from the second device, a plurality of estimates of a position of the second device relative the first device using a plurality of position estimation algorithms; determining, based on the plurality of estimates of the position of the second device relative the first device, whether the second device is located in the medical room of the medical facility in which the first device is located; and in accordance with determining that the second device is located in the same medical room as the first device, establishing a wireless communication connection with the second device.

## Description

### CROSS-REFERENCE TO RELATED APPLICATION

This application claims the benefit of U.S. Provisional Application No. 63/754,402 filed on February 5, 2025, the entire content of which is incorporated herein by reference for all purposes.

### FIELD

The present disclosure relates generally to medical device wireless communication and, more specifically, to wireless communication of medical data.

### BACKGROUND

Medical environments typically include multiple medical devices that are communicatively connected to one another, such as for control or for communicating medical data. For example, a control device may be communicatively connected to a medical imaging system to provide control commands to the medical imaging system and to receive medical imaging data from the medical imaging data system for routing to a display device or for performing image processing. Medical environments often utilize mobility for medical devices so that medical devices can be moved from room to room as needed. For example, a particular imaging system may be provided on a mobile cart so that the imaging system can be moved from a location where the imaging system is not being utilized at a given time to a location where the imaging system is needed, such as an operating room in which a surgical procedure is to be conducted that requires the medical imaging system. Some mobile medical devices have wireless communication capability, avoiding the need to connect and route cumbersome data cables. A mobile wireless medical device moved into a given room of a medical facility may need to be communicatively connected with other devices in the room. This may require a user to perform various manual steps to connect devices, which may be time consuming, prone to error, and require specialized training.

### SUMMARY

According to an aspect, systems and methods include wirelessly connecting devices based on determining whether the devices are in the same room of a medical facility. One or both of a pair of devices that are within wireless communication range of one another may determine multiple estimates of their positions relative to one another based on one or more wireless signals transmitted between them. The multiple estimates of relative position may be determined using different relative position estimation algorithms that are affected differently by transmission of the one or more wireless signals through barriers defining a room, such as walls, ceilings, floors, windows, and doors. One or both of the devices may compare different relative position estimates to determine whether the devices are in the same room. If a determination is made that the devices are in the same room, the devices may establish a communication link with one another (with or without requiring user confirmation). If the determination is made that the devices are not in the same room, the devices may not establish the communication link with one another (optionally, unless a user overrides the determination). As such, devices, such as mobile devices, can be communicatively connected with minimal user interaction.

According to an aspect, a method for establishing wireless communication between devices in a medical facility includes, at a first device located in a medical room of the medical facility: receiving at least one wireless signal from a second device; determining, based on the at least one wireless signal received from the second device, a plurality of estimates of a position of the second device relative the first device using a plurality of position estimation algorithms; determining, based on the plurality of estimates of the position of the second device relative the first device, whether the second device is located in the medical room of the medical facility in which the first device is located; and in accordance with determining that the second device is located in the same medical room as the first device, establishing a wireless communication connection with the second device.

The plurality of position estimation algorithms may include at least one distance estimation algorithm or at least one direction estimation algorithm. The plurality of position estimation algorithms may include a first distance estimation algorithm that is based on time-of-flight and a second distance estimation algorithm that is based on signal strength.

Determining whether the second device is located in the same medical room as the first device may include comparing the plurality of estimates of the position of the second device relative the first device; and determining a confidence in a determination of whether the second device is located in the same medical room as the first device based on the comparison of the plurality of estimates of the position of the second device relative to the first device. Determining the confidence in a determination of whether the second device is located in the same medical room as the first device may include evaluating a result of the comparison of the plurality of estimates of the position of the second device relative to the first device based on at least one threshold. The at least one threshold may be associated with wireless signal propagation through at least one type of obstacle. The at least one type of obstacle may include a wall, a floor, a door, or a window. The at least one threshold may be associated with a risk level assigned to at least one of the first device and the second device, the risk level being associated with a risk posed by the at least one of the first device and the second device being communicatively connected to a device in a different medical room. The at least one threshold may have been determined during a set-up of the first device in the medical room. The at least one threshold may be updated over time based on user input associated with confirmation of whether devices are located in the same medical room.

The method may include, prior to establishing the wireless communication connection with the second device, requesting user confirmation to establish the wireless communication connection with the second device. The user confirmation may be requested based on a risk level associated with at least one of the first device and the second device.

The method may include, after establishing the wireless communication connection with the second device, periodically determining whether the second device is in the same medical room as the first device. The method may include determining that the second device is no longer in the same medical room as the first device and breaking the wireless communication connection with the second device. The method may include, prior to breaking the wireless communication connection with the second device, receiving user confirmation to break the wireless communication connection with the second device. The method may include requesting the user confirmation based on a risk level associated with at least one of the first device and the second device.

The first device or the second device may establish simultaneous wireless communication connections with a plurality of devices. A controller may be connected to one of the first device and the second device via a wired connection, the controller configured to send control commands via the wireless communication connection to the other of the first device and the second device.

The method may include, at the first device: receiving at least one wireless signal from a third device; determining, based on the at least one wireless signal received from the third device, a at least one estimate of a position of the third device relative the first device using at least one of the plurality of position estimation algorithms; and determining a position within the medical room of the first device based at least in part on the at least one estimate of the position of the second device relative the first device and at least one of the plurality of estimates of the position of the third device relative the first device.

According to an aspect, a system includes a plurality of devices configured for communicating wirelessly, the plurality of devices comprising a first device that is configured to: receive at least one wireless signal from a second device; determine, based on the at least one wireless signal received from the second device, a plurality of estimates of a position of the second device relative the first device using a plurality of position estimation algorithms; determine, based on the plurality of estimates of the position of the second device relative the first device, whether the second device is located in the same medical room of a medical facility as the first device; and in accordance with determining that the second device is located in the same medical room as the first device, establish a wireless communication connection with the second device.

The plurality of position estimation algorithms may include at least one distance estimation algorithm or at least one direction estimation algorithm. The plurality of position estimation algorithms may include a first distance estimation algorithm that is based on time-of-flight and a second distance estimation algorithm that is based on signal strength.

Determining whether the second device is located in the same medical room as the first device may include comparing the plurality of estimates of the position of the second device relative the first device; and determining a confidence in a determination of whether the second device is located in the same medical room as the first device based on the comparison of the plurality of estimates of the position of the second device relative to the first device. Determining the confidence in a determination of whether the second device is located in the same medical room as the first device may include evaluating a result of the comparison of the plurality of estimates of the position of the second device relative to the first device based on at least one threshold. The at least one threshold may be associated with wireless signal propagation through at least one type of obstacle. The at least one type of obstacle may include a wall, a floor, a door, or a window. The at least one threshold may be associated with a risk level assigned to at least one of the first device and the second device, the risk level being associated with a risk posed by the at least one of the first device and the second device being communicatively connected to a device in a different medical room. The at least one threshold may have been determined during a set-up of the first device in the medical room. The at least one threshold may be updated over time based on user input associated with confirmation of whether devices are located in the same medical room.

The first device may be configured to, prior to establishing the wireless communication connection with the second device, request user confirmation to establish the wireless communication connection with the second device. The first device may be configured to request the user confirmation based on a risk level associated with at least one of the first device and the second device. The first device may be configured to, after establishing the wireless communication connection with the second device, periodically determine whether the second device is in the same medical room as the first device. The first device may be configured to break the wireless communication connection with the second device in response to determining that the second device is not in the same medical room as the first device. The first device may be configured to, prior to breaking the wireless communication connection with the second device, receive user confirmation to break the wireless communication connection with the second device. The first device may be configured to request the user confirmation based on a risk level associated with at least one of the first device and the second device.

At least one of the first device and the second device may be configured to establish simultaneous wireless communication connections with a plurality of devices. The system may include a controller that is connected to one of the first device and the second device via a wired connection, the controller configured to send control commands via the wireless communication connection to the other of the first device and the second device.

The first device is configured to: receive at least one wireless signal from a third device; determine, based on the at least one wireless signal received from the third device, a at least one estimate of a position of the third device relative the first device using at least one of the plurality of position estimation algorithms; and determine a position within the medical room of the first device based at least in part on the at least one estimate of the position of the second device relative the first device and at least one of the plurality of estimates of the position of the third device relative the first device.

According to an aspect, a computer program product, or a non-transitory computer-readable medium include one or more programs for execution by one or more processors of a first device, the one or more programs including instructions that when executed by the one or more processors cause the first device to perform any of the methods described above.

It will be appreciated that any of the variations, aspects, features, and options described in view of the systems applies equally to the methods and vice versa. It will also be clear that any one or more of the above variations, aspects, features, and options can be combined.

### BRIEF DESCRIPTION OF THE DRAWINGS

The invention will now be described, by way of example only, with reference to the accompanying drawings, in which:
FIG. 1 illustrates an exemplary medical facility that includes one or more wireless devices that are configured to automatically determine whether other devices within wireless communication range are in the same medical room to facilitate wireless connection of the devices without burdensome manual connection steps;
FIG. 2A illustrates an exemplary notification that may be displayed to inform a user that a device can be connected and to enable the user to confirm the connection to the device;
FIG. 2B illustrates an exemplary notification that may be displayed for enabling a user to connect to a device that has been determined to be in another room;
FIG. 3 is a functional block diagram of a device that is configured for determining whether other devices that are within wireless communication range are in the same room and establishing communication with one or more devices that are determined to be in the same room;
FIG. 4 illustrates an exemplary method for establishing wireless communication between devices in a medical facility, according to the principles described herein;
FIG. 5 illustrates an exemplary visualization that can be displayed on a display to a user to guide the user in placement of various devices in a medical room; and
FIG. 6 illustrates an exemplary computing system.

### DETAILED DESCRIPTION

Reference will now be made in detail to implementations and examples of various aspects and variations of the invention, examples of which are illustrated in the accompanying drawings. Various devices, systems, and methods are described herein. Although at least two variations of the devices, systems, and methods are described, other variations may include aspects of the devices, systems, and methods described herein combined in any suitable manner having combinations of all or some of the aspects described. Examples will now be described more fully hereinafter with reference to the accompanying drawings; however, they may be embodied in different forms and should not be construed as limited to the examples set forth herein. Rather, these examples are provided so that this disclosure will be thorough and complete, and will fully convey exemplary implementations to those skilled in the art.

According to various aspects, systems, devices, and methods include establishing wireless communication between devices in a medical room of a medical facility. One or more of the devices may be a mobile device that may be moved from one room to another according to the needs of medical personnel in the medical facility. Upon being moved into a given medical room, the mobile device may be connected to other devices in the medical room using the principles described herein. The mobile device may be connected with no user involvement or with minimal user involvement, such as a confirmation from a user that the mobile device should be connected to one or more other devices. As such, the systems, devices, and methods described herein can facilitate ad hoc connection of devices with wireless communication capability with minimal or no user interaction, avoiding the often time-consuming and error-prone process of manually connecting wireless devices in a medical facility.

According to various aspects, a first device located in a given medical room of a medical facility receives at least one wireless signal from a second device that may or may not also be located in the same medical room. This signal may be broadcast by the second device or transmitted in response to a request from the first device. The signal can be associated with various wireless communication protocols such as ultra-wideband, Wi-Fi, Bluetooth, RFID, or ZigBee. The signal may include one or more pulses across a wide spectrum of frequencies. The first device determines a plurality of estimates of the second device's position relative to the first device using multiple position estimation algorithms. These algorithms may include distance estimation algorithms, such as time-of-flight or received signal strength indicator (RSSI) algorithms. The first device then determines whether the second device is in the same medical room by comparing the plurality of position estimates. This comparison may include determining differences between estimates and assessing whether these differences are within a predefined threshold. The differences can be absolute (e.g., meters or feet) or relative (e.g., percentage difference). The first device may determine that the second device is in the same room if the differences are below the threshold. For example, a relative position estimate based on time-of-flight and a relative position estimate based on signal strength may be similar when the wireless signals received from the second device pass through air but significantly dissimilar when the wireless signals have passed through an obstacle associated with a boundary of a medical room, such as a window, wall, door, floor, or ceiling. By comparing relative position estimates generated using different algorithms, the first device can determine whether signals from the second device have passed through a room boundary, and therefore, whether the second device is on the other side of a room boundary, without the first device having to determine an actual location of the second device.

The first device may determine a confidence in its determination of whether the second device is located in the same medical room, which can be based on the extent of differences between estimates, variability over time, or consistency with determinations made by other devices in the room. For example, if the position estimates from different algorithms are consistent and show minimal variability over time, the confidence level in the determination may be high. Conversely, if there is significant variability or inconsistency, the confidence level may be low.

If the second device is determined to be in the same medical room, the first device may establish a wireless communication connection with the second device using a suitable wireless protocol, such as ultra-wideband, Wi-Fi, Bluetooth, RFID, or ZigBee, which may include exchanging necessary information, such as passwords or passkeys, for establishing the connection. User confirmation to connect to the second device may be requested based on risk levels associated with one or both of the devices, particularly if the devices are involved in patient care. For example, high-risk devices like controllers that control patient treatment devices, like fluid management devices or patient imaging devices, may require user confirmation before establishing a connection, whereas devices like displays or data storage devices may not. Optionally, the confidence of the first device in its determination of whether the second device is in the same medical room is a factor in whether to request user confirmation. If the confidence level is low, user confirmation may be requested to ensure the safety and accuracy of the connection.

The first device may periodically re-evaluate whether the second device is still in the same medical room to account for potential movement of the first device and/or the second device. If a determination is made that the devices are no longer in the same room, the connection may be terminated, optionally with user confirmation. This periodic check ensures that mobile devices that have been moved out of the room do not remain connected.

The systems, devices, and method described herein provide a robust and flexible approach for establishing wireless communication between devices in a medical facility with little or no user interaction. By leveraging multiple position estimation algorithms, the systems, devices, and methods enable as few as two devices to determine whether the devices are in the same medical room without requiring highly accurate position estimates. The inclusion of user confirmation based on risk levels and confidence levels further enhances the safety and reliability of the system, making it suitable for critical medical applications.

In the following description, it is to be understood that the singular forms "a," "an," and "the" used in the following description are intended to include the plural forms as well, unless the context clearly indicates otherwise. It is also to be understood that the term "and/or" as used herein refers to and encompasses any and all possible combinations of one or more of the associated listed items. It is further to be understood that the terms "includes," "including," "comprises," and/or "comprising," when used herein, specify the presence of stated features, integers, steps, operations, elements, components, and/or units but do not preclude the presence or addition of one or more other features, integers, steps, operations, elements, components, units, and/or groups thereof.

Certain aspects of the present disclosure include process steps and instructions described herein in the form of an algorithm. It should be noted that the process steps and instructions of the present disclosure could be embodied in software, firmware, or hardware and, when embodied in software, could be downloaded to reside on and be operated from different platforms used by a variety of operating systems. Unless specifically stated otherwise, as apparent from the following discussion, it is appreciated that, throughout the description, discussions utilizing terms such as "processing," "computing," "calculating," "determining," "displaying," "generating," or the like, refer to the action and processes of a computer system, or similar electronic computing device, that manipulates and transforms data represented as physical (electronic) quantities within the computer system memories or registers or other such information storage, transmission, or display devices.

The present disclosure, in some aspects, also relates to devices or systems for performing the operations herein. The devices or systems may be specially constructed for the required purposes, may comprise a general-purpose computer selectively activated or reconfigured by a computer program stored in the computer, or may include any combination thereof. Computer instructions for performing the operations herein can be stored in any combination of non-transitory, computer-readable storage media, such as, but not limited to, any type of disk, including floppy disks, USB flash drives, external hard drives, optical disks, CD-ROMs, magnetic-optical disks, read-only memories (ROMs), random access memories (RAMs), EPROMs, EEPROMs, magnetic or optical cards, or any type of media suitable for storing electronic instructions, and each coupled to a computer system bus. One or more instructions for performing the operations herein may be implemented in or executed by one or more Application Specific Integrated Circuits (ASICs), Field Programmable Gate Arrays (FPGAs), Digital Signal Processing units (DSPs), Graphics Processing Units (GPUs), or Central Processing Units (CPUs). Furthermore, the computers referred to herein may include a single processor or may be architectures employing multiple processor designs for increased computing capability.

The methods, devices, and systems described herein are not inherently related to any particular computer or other apparatus. Various general-purpose systems may also be used with programs in accordance with the teachings herein, or it may prove convenient to construct a more specialized apparatus to perform the required method steps. The required structure for a variety of these systems will appear from the description below. In addition, the present invention is not described with reference to any particular programming language. It will be appreciated that a variety of programming languages may be used to implement the teachings of the present invention as described herein.

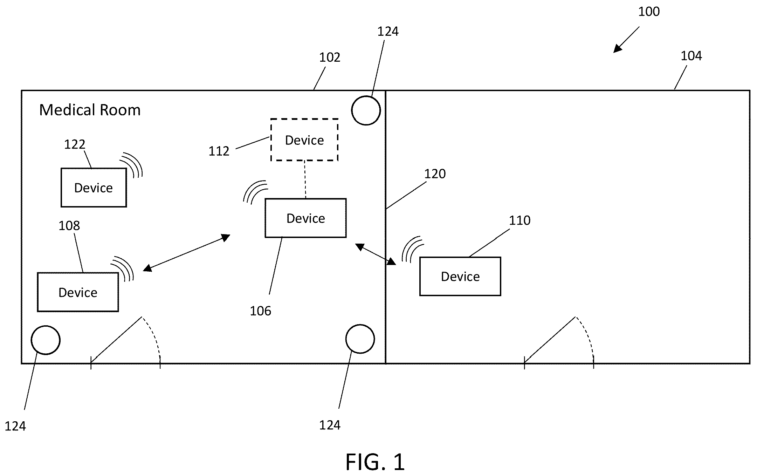

FIG. 1 illustrates an exemplary medical facility 100 that includes one or more wireless devices that are configured to automatically determine whether other devices within wireless communication range are in the same medical room to facilitate wireless connection of the devices without burdensome manual connection steps. By automatically determining whether another device is in the same medical room, the devices can establish a communication link with little to no user involvement while avoiding linking devices that are not in the same medical room. This is particularly useful in a medical environment that often includes multiple of the same types of devices, many of which are mobile and are moved from location to location within the medical environment.

In the illustrated example, medical facility 100 includes a medical room 102 and an area 104 that is outside of the medical room 102, such as an adjacent medical room, a hallway, a nurse's station, a storage room, an area above the medical room 102, or an area below the medical room 102. The medical room 102 can be an operating room, an examination room, a medical imaging room, a laboratory, or any other enclosed space in a medical facility within which medical devices are used. A first device 106 and a second device 108 are located in the medical room 102. The first device 106 and the second device 108 are configured for wireless communication and are within wireless communication range of each other, and thus, can wirelessly communicate with each other and other devices. The first device 106 and the second device 108 can each be any type of electronic device that sends data to and/or receives data from any other electronic device. For example, the first device 106 and/or the second device 108 may be a controller, a network communication device, an imaging device, a fluid management device, a display device, an image processing device, a surgical tool controller, a patient support device, or any other device used within a medical facility that communicates with other devices.

In some examples, the first device 106 may be a network connection device that is configured for establishing wireless communication connections with devices, such as the second device 108, to enable those devices to communicate with other devices. For example, the first device 106 may be a wireless communication hub, router, or access point configured to wirelessly connect with the second device 108 to enable the second device 108 to communicate with a device 112 that is communicatively connected (wirelessly or via a wired connection) to the first device 106. The device 112 may be, for example, a controller configured to control other devices in the medical room 102, and the first device 106 may enable connection of the controller to wireless devices such as the second device 108. The second device 108 may be a device used in treatment of a patient, such as a medical imager for imaging a patient during a medical procedure, a fluid management device for delivering fluid to and/or removing fluid from a treatment area during a medical procedure, a monitoring device configured for monitoring a patient during a medical procedure, an insufflation device, a display device configured for displaying treatment information to medical personnel during a procedure, or an image processing device configured to process image data, such as for providing image enhancement or for medical procedure guidance. As used herein, the term "device" encompasses not only a single component, such as a network router or a display device, but also a collection of components that interoperate to provide particular functionality. For example, an endoscopic imaging device may include a collection of components that work together to capture images for display to medical personnel, such as a camera head for generating pixel data based on light received from a scene, a camera control unit for controlling the camera head to generate the pixel data and/or to generate images from the pixel data, a light generator for generating light for illuminating the scene, a display for displaying the images, and a wireless transceiver for transmitting images to one or more other devices and/or receiving control commands from one or more other devices.

The first device 106 and/or the second device 108 may be a mobile device that is configured to be readily moved from location to location in the medical facility. For example, the second device 108 may include a mobile cart or other mobile chassis that enables the second device 108 to be wheeled between medical room 102 and one or more other locations in the medical facility, such as area 104. Optionally, the first device 106 or the second device 108 is a fixed device that, in general usage, is not intended to be moved from location to location within the medical facility. For example, the first device 106 may be a networking device that is mounted in a fixed position (e.g., to a ceiling) in the medical room 102.

One or both of the first device 106 and the second device 108 are configured to determine whether the other of the first device 106 and the second device 108 is in the same medical room. The first device 106 and the second device 108 transmit and receive wireless signals between one another, and at least one of the first device 106 and the second device 108 determines a plurality of estimates of a relative position of the other of the first device 106 and the second device 108 based on the wireless signals. The plurality of relative position estimates are determined using different algorithms that have different accuracies depending on whether a given wireless signal or set of wireless signals passes through a barrier, such as a wall, window, door, ceiling, or floor. Relatively large disparities between relative position estimates provided by different algorithms may be used to determine that the first device 106 and the second device 108 are not in the same room. For example, since the speed of travel of a wireless signal is not affected by traversal through a wall but the strength of the wireless signal generally is, a position estimation algorithm that is based on time-of-flight and a position estimation algorithm that is based on signal strength may provide position estimates that differ, or differ to a greater degree, when the wireless signal passes through a wall. The first device 106 and/or the second device 108 utilize potential disparities between different relative position estimation algorithms to determine whether the first device 106 and the second device 108 are in the same room.

The arrangement depicted in FIG. 1 illustrates an example of how different relative position estimation algorithms can be used to determine whether devices are in the same medical room. The first device 106 may receive wireless signals from the second device 108, which is in the same medical room 102, and from a third device 110 that is in the area 104 outside the medical room 102. The wireless signals from the second device 108 do not pass through any walls, windows, doors, ceiling, or floor of the medical room 102 to reach the first device 106. However, wireless signals from the third device 110 pass through a wall 120 of the medical room 102 to reach the first device 106. The first device 106 may generate estimates for the position of the third device 110 relative to the first device 106 using multiple different algorithms. The multiple different algorithms may produce estimates that differ to a relatively significant degree when a signal passes through barriers associated with extents of a room, such as walls, windows, doors, ceilings, and floors. For example, the first device 106 may determine (1) a first estimate of the distance between the first device 106 and the third device 110 using a time-of-flight algorithm that determines how far a signal has traveled based on how long it takes for the signal to travel between the first device 106 and the third device 110, and (2) a second estimate of the distance between the first device 106 and the third device 110 using a signal strength algorithm that determines how far a signal has traveled based on its power loss. The wall 120 does not reduce the speed of the signal but does reduce the power of the signal, and thus, the distance estimates provided by the two algorithms differ. For example, the signal strength algorithm may provide a longer distance estimate than the time-of-flight algorithm due to the attenuation of the signal by the wall 120. The first device 106 may compare the two distance estimates and determine based on the comparison that the third device 110 is not in the same medical room because the two distance estimates differ or differ by a sufficient, or threshold, amount.

The first device 106 may determine distance estimates for the second device 108 using the same algorithms. The distance estimates for the second device 108 do not differ or minimally differ because the signals transmitted between the first device 106 and the second device 108 do not pass through the walls, windows, doors, ceiling, or floor of the medical room 102. Note that in contrast to triangulation techniques, the first device 106 may not base its determination of whether the second device 108 or the third device 110 is in the same medical room on how far the second device 108 or the third device 110 is from the first device 106, but on whether different distance estimates sufficiently differ. This approach provides an advantage over triangulation techniques in that it enables the first device 106 to determine whether a given device is in the same room without requiring distance measurements to multiple different wireless transceivers, as required for triangulation techniques, which reduces complexity and cost. Additionally, while certain relative position estimation algorithms, such as one based on signal strength, could be used alone to determine whether a given signal has traveled through walls, such an approach may have limited application due to sensitivities to the configurations of rooms (e.g., the types of walls, doors, windows, floors, etc.), the locations of devices within a room, and other factors. For example, a signal that travels a relatively short distance from one room to another through a relatively low-density wall may have a similar drop in signal strength as a signal that travels across a large room, and therefore, a device that relies only on signal strength to determine whether another device is in the same room may falsely determine that the device on the other side of the wall is in the same room or determine that the device across the room is in a different room. By using multiple different relative position estimation algorithms, the devices describe herein may be more robust to different environments and scenarios.

The discussion above often refers to the first device 106 as determining whether the second device 108 and the third device 110 are in the same medical room as the first device 106. Additionally, or alternatively, the second device 108 and/or the third device 110 may determine whether the first device 106 is in the same medical room. Moreover, although three wireless devices are described above, other examples may include wireless pairing across any number of devices.

Based on the determination that the first device 106 and the second device 108 are in the same medical room, the first device 106 and/or the second device 108 may automatically establish a communication link between the first device 106 and the second device 108. For example, the first device 106 may automatically establish a communication link with the second device 108 such that data can be communicated between them. Optionally, the first device 106 and/or the second device 108 may automatically initiate a process for establishing the communication link, but the link establishment process may include one or more steps that involve user input. For example, initiation of the process for establishing the communication link may include the first device 106 and/or the second device 108 automatically providing a notification to a user that the other device is in the same medical room and requesting confirmation of whether to establish a connection.

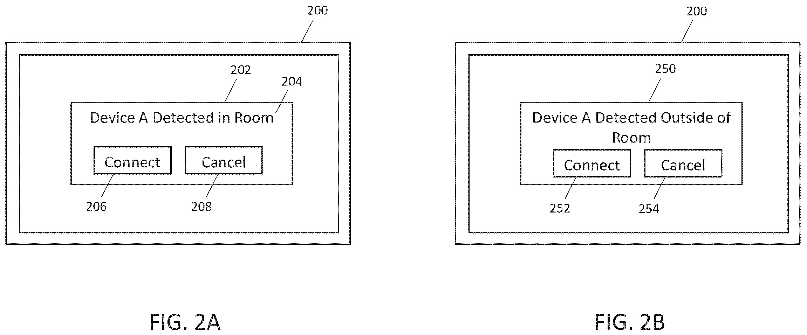

FIG. 2A illustrates an example notification that may be displayed on a display 200 to inform the user that a device can be connected and to enable the user to confirm the connection to the device. The display 200 may be a display of the first device 106 or the second device 108 or a display communicatively connected to the first device 106 or the second device 108 (e.g., a display of device 112). The notification 202 may include an indication 204 that another device has been determined to be in the same room. The notification 202 may include a graphical component 206 that a user may use to confirm connection to the device and, optionally, a graphical component 208 that a user may use to cancel connection to the device. Optionally, devices (e.g., the first device 106 and the second device 108) communicate identifying information, such as a device type, a device given name, or a device serial number, and the notification 202 includes the identifying information to enable a user to identify which device may be connected. Optionally, notification 202 may be provided on displays associated with both the first device 106 and the second device 108, and user confirmation may be required on both devices before a communication link is established.

A notification such as notification 202 may be provided any time that a device is determined to be in the same medical room in order to obtain user confirmation of connection to the device. Alternatively, the notification 202 may be displayed for connection to some devices but not others. Whether to display the notification 202 may depend on a risk level associated with a given device. The risk level may be associated with risk to a patient. For example, a device that is associated with patient treatment, such as a fluid management device configured for delivering fluid to a patient during a medical procedure, may have a relatively high risk level since inadvertent control of the fluid management device may harm the patient. As such, notification 202 may be displayed to require user confirmation to connect to the fluid management device. A low risk level device may be a device that is not involved directly in patient treatment. For example, a display device may be a low-risk device, and connection with the display device may occur automatically without notification 202 being displayed.

Optionally, a user may be provided with the ability to instruct connection to a device despite a determination being made that the device is not in the same room. For example, the first device 106 may provide a notification to a user that the third device 110 was detected but is not in the same room and may provide the user with the option to connect to the third device 110 anyway. FIG. 2B illustrates an exemplary notification 250 that may be displayed on display 200 for enabling a user to connect to a device that has been determined to be in another room. Notification 250 may include an indication that a device has been detected and determined to be in another room. The notification 250 may include a graphical component 252 for a user to instruct connection to the device and a graphical component 254 for a user to deny connection to the device.

Upon user confirmation to connect to a device, an indicator may be activated that indicates that a connection process has been initiated and/or that a connection to the device is complete. The indicator may be a graphical indicator, such as a notification displayed on display 200 that informs the user that a connection is being established and/or that a connection has been established. Additionally, or alternatively, the indicator may include a light of the device that received the user confirmation to connect to the other device and/or a light of the other device. Optionally, a light indicator may have a color associated with a particular room. For example, a light of a device may be configured to illuminate in multiple different colors, and the color provided by the device when connecting to another device may depend on the room in which the devices are located. Additionally, or alternatively, the indicator may include an audible indicator, such as a chime, emitted from a speaker of the device that received the user confirmation to connect to the other device and/or of the other device.

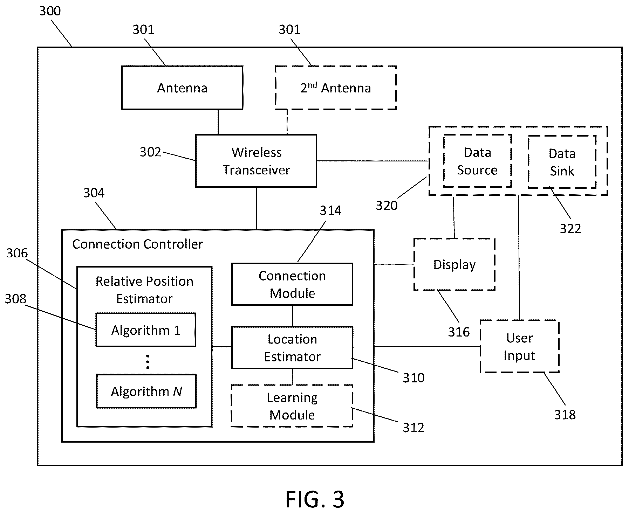

FIG. 3 is a functional block diagram of a device 300 that is configured for determining whether other devices that are within wireless communication range are in the same room as device 300 and establishing communication with one or more devices that are determined to be in the same room. Device 300 can be used for any of the devices described above, including first device 106, second device 108, and/or third device 110. The blocks of FIG. 3 indicate functions of device 300 and may be implemented in hardware, software, or a combination of hardware and software.

Device 300 includes at least one wireless transceiver 302 for processing wireless signals received via one or more antennas 301 and generating signals for transmitting via the one or more antennas 301. The wireless transceiver 302 may obtain information from processed signals that is useable for determining whether another device is in the same room. For example, wireless transceiver 302 may obtain time information encoded in a signal that can be used to determine when the signal was transmitted, and/or wireless transceiver 302 may determine a signal strength of a given signal. Optionally, wireless transceiver 302 may determine the timing of arrival of a given signal at two different antennas 301, which may be used for determining a direction of arrival of the signal. Wireless transceiver 302 may encode onto generated signals information that is useable for determining whether another device is in the same room and/or for enabling another device to determine whether device 300 is in the same room. For example, wireless transceiver 302 may encode timing information related to when a given signal is transmitted and/or may encode signal strength information indicating power of a signal transmitted by the one or more antennas 301. Wireless transceiver 302 may obtain information associated with identifying a device from which wireless signals are received. For example, wireless transceiver 302 may extract device identifier, such as a serial number, a device name, a device type, a MAC address, an SSID, or any other identifying information, that is encoded in one or more received signals. Wireless transceiver 302 may be configured to implement one or more wireless communication protocols, such as ultra-wideband, Wi-Fi, Bluetooth, RFID, ZigBee, or any other wireless communication protocol. Optionally, device 300 includes multiple transceivers 302 for implementing different wireless communication protocols. As such, wireless transceiver 302 can be a single transceiver implementing one or more wireless communication protocols or multiple transceivers implementing multiple different wireless communication protocols.

Connection controller 304 may control determination of whether another device is in the same medical room and may control the establishment of a data communication link with a device has been determined to be in the same medical room. Connection controller 304 may receive information obtained by wireless transceiver 302 that connection controller 304 uses to determine whether to connect to another device. The information may include information associated with the relative position of the other device, such as time-of-flight information and/or signal strength information. The information may include, for example, one or more identifiers indicating the device and/or the type of device signals from which signals were received. The information may include an indicator that indicates to the connection controller 304 that the other device is a device for which connection controller 304 should make a determination regarding presence in the same medical room. For example, devices that are configured for automatic connection, according to the principles described herein, may broadcast signals that include an indicator (e.g., a specific data block stored in memory of the device) indicating to other devices that they implement an automatic connection protocol, and connection controller 304 may determine whether the information obtained by the wireless transceiver 302 from one or more signals received from a given device has the automatic connection protocol indicator. If not, the connection controller 304 may not further process information from the one or more signals. Optionally, the wireless transceiver 302 itself may look for the automatic connection protocol indicator and may provide information to the connection controller 304 only if the indicator is identified.

Device 300 may use information received in one or more signals to determine whether the signals are associated with a device or a type of device to which device 300 wants to connect. For example, device 300 may be configured to connect to certain devices and/or certain types of devices and device 300 may ignore signals that are not from devices or types of devices to which device 300 is configured to connect. This approach may be useful in enabling different types of devices in the same medical room to connect to some but not all other devices in the same medical room. For example, display devices may seek to connect to video sources and may look for video source identifiers in received signals, and/or lighting control devices may seek to connect to lighting devices and may look for lighting device identifiers in received signals. In some examples, a given medical room may have different fixed wireless communication devices for connecting to different types of devices that move into the medical room, and device 300 may be configured to connect to the fixed wireless communication device to which device 300 corresponds. For example, one fixed wireless communication device may be dedicated to receiving video from one or more video sources (e.g., an imaging system) and another fixed wireless communication device may be dedicated to transmitting video to one or more video sinks (e.g., a display), and device 300 may connect to one or the other of the fixed wireless communication devices based on whether device 300 is a video source or a video sink. Optionally, device 300 may be configured to connect to a device that is dedicated to device control, such as a wireless hub.

Connection controller 304 may control transmission of information to other devices for use in determining whether the devices are in the same room. For example, connection controller 304 may control the broadcasting of information that may be used by other devices in implementing the features described herein. The information may include any of the information described above, such as one or more identifiers identifying device 300 and/or identifying the type of device 300 and/or one or more indicators indicating that device 300 implements an automatic connection protocol. The information may include information for determining relative position estimates. For example, the information may include time-of-flight information (e.g., a time of departure of a signal) and/or signal strength information. Connection controller 304 may control periodic broadcasting of information that lets other devices that come into wireless communication range know that device 300 is present. Connection controller 304 may control transmission of information in response to receiving a signal from another device. For example, connection controller 304 may respond to device 300 receiving a signal from another device by controlling the transmission to the other device of time-of- flight information and/or information that requests time-of-flight information from the other device.

Connection controller 304 may include a relative position estimator 306 that uses information obtained by wireless transceiver 302 to generate a plurality of different estimates of a position of a device from which signals are received relative to device 300. Relative position estimator 306 may implement a plurality of different relative position estimation algorithms 308 to determine the different relative position estimates. An example of a relative position estimation algorithm 308 that may be implemented by relative position estimator 306 is a time-of-flight algorithm that uses timing information associated with the time that it takes one or more wireless signals to travel between devices to determine the distance between the devices. Another example of a relative position estimation algorithm 308 that may be implemented by relative position estimator 306 is a signal strength algorithm that estimates the distance between devices based on power loss-the difference between the power of the signal when it was transmitted by a device and the power of the signal when it is received by device 300. Another relative position estimation algorithm 308 that may be implemented by relative position estimator 306 is a direction of arrival algorithm that estimates the position of a device relative to device 300 in terms of the direction from which signals from the device are received. The relative position estimator 306 may use timing information related to when a signal was received at two different antennas 301 (e.g., obtained by transceiver 302) and predetermined distance between the two different antennas 301 to determine a direction of arrival of the signal and, thereby, the direction that the device from which the signal was received is relative to the device 300.

The plurality of different relative position estimates from the relative position estimator 306 may be used by a location estimator 310 to determine whether the device from which signals were received is located in the same room as device 300. Location estimator 310 may compare different relative position estimates and may determine whether a device is in the same room as device 300 based on whether, and/or how much, the relative position estimates differ. A determination of whether a device is in the same room may be based on one or more thresholds. The one or more thresholds may include an absolute threshold, such as a threshold difference in distance estimates. For example, a device may be determined to be in the same room if two different distance estimates differ by less than a threshold amount (e.g., one meter). A threshold may correspond to a relative difference between distance estimates. For example, a device may be determined to be in the same room if two different distance estimates differ by less than a threshold percentage (e.g., ten percent).

One or more thresholds used to determine whether a device is in the same room as device 300 may be configurable. For example, during a setup process associated with installation of device 300 in a medical room, one or more setup tests may be conducted by an installer to determine what threshold provides desired results. The setup process may enable one or more thresholds to be set that account for the specific attributes of a given room. As such, different rooms may be associated with different thresholds, which may account for differences in how a room is constructed (e.g., the density of the walls, the number and size of doors and windows, the size of the room), the contents of the room, and any other factors. As such, at least one threshold may be associated with wireless signal propagation through at least one type of obstacle associated with a given medical room. Optionally, a default threshold may be used (which may or may not be updatable during a setup process or other configuration process) that is based on wireless signal propagation through obstacles commonly associated with delineating medical rooms. For example, one or more default thresholds may be associated with wireless signal propagation through walls made of dry wall, windows made of glass, doors made of wood, etc. Device 300 may store several default thresholds associated with different types of boundaries, such as different types of walls (e.g., dry wall versus concrete block), and at least one of the default thresholds may be set during a setup process according to the particular configuration of a given room. For example, a setup process may include the installer making selections defining the types of boundaries of a given room, and device 300 may select a suitable threshold based on the selected boundaries.

Optionally, different thresholds are used for different types of devices. For example, a threshold that is more difficult to meet (e.g., a threshold that requires relative position estimates to be more similar) may be used for a higher-risk-level device, such as a device directly involved in patient treatment, and a threshold that is easier to meet may be used for a lower-risk-level device, such as a display device. The type and/or risk level of a given device may be included in information received from the device (e.g., obtained by wireless transceiver 302 from one or more signals) and/or may be stored in memory of device 300 (or in a server communicatively connected to device 300) in association with an identifier of the device.

One or more thresholds may be automatically updated over time based on the history of the device 300 connecting to other devices. For example, device 300 may update a threshold in response to a user overriding (e.g., via notification 250 of FIG. 2) a determination that another device is not in the same medical room. The device 300 may update the threshold to a value that would have resulted in the other device being determined to be in the same medical room. The connection controller 304 may include a learning module 312 that may adjust one or more thresholds used by location estimator 310 based on the experience of the device 300 in connecting to other devices. Learning module 312 may adjust one or more thresholds based on a user instructing connection with a device that was determined to not be in the same room as device 300. Learning module 312 may adjust the one or more thresholds based on the type of the device. For example, different thresholds may be used for different types of devices (e.g., different threshold associated with different risk levels), and learning module 312 may adjust a threshold associated with the type of device that the user instructed to connect to, while leaving one or more thresholds associated with other types of devices unchanged. Learning module 312 may use one or more machine learning modules to automatically update one or more thresholds based on experience of the device 300 in connecting to other devices.

Connection controller 304 may include a connection module 314 that controls the establishment of a data communication link between device 300 and a device that has been determined to be in the same medical room as device 300. Connection module 314 may be configured to establish the data communication link according to one or more wireless communication protocols, such as any of the wireless communication protocols discussed herein. Connection module 314 may utilize the same wireless transceiver 302 that was used in determining whether a device is in the same medical room or may use a different wireless transceiver. For example, device 300 may include a first wireless transceiver that is configured for obtaining information useable for determining whether another device is in the same medical room and a second wireless transceiver for establishing a data communication link with the other device.

Optionally, connection module 314 enables a second device that is communicatively connected to device 300 to establish a wireless communication connection with a device that has been determined to be in the same medical room. As such, device 300 may be used to facilitate the establishment of wireless communication connections between other devices and not with itself (or not only with itself). For example, with reference to FIG. 1, the first device 106 configured according to device 300 may determine that the second device 108 is in medical room 102 and may obtain wireless communication connection information (e.g., a device name or SSID) from the second device 108. The connection module 314 of the first device 106 may then control the sending of this information to device 112 to enable device 112 to establish a direct wireless communication connection with the second device 108 such that data is exchanged between the second device 108 and device 112 without involvement of the first device 106. With this functionality, device 300 may enable the implementation of the automatic connection methods described herein with a device that does not have the device location determination capabilities described herein.

Device 300 may include one or more displays 316. The one or more displays 316 may display to a user one or more notifications associated with device 300 connecting to a device that is within wireless communication range (e.g., notification 202 or notification 250). Device 300 may include one or more user input devices 318, such as a keyboard, a mouse, a touch input device (e.g., integrated with a display 316 as a touchscreen), a voice command device, and/or a gesture recognition device. The user input device 318 may receive user inputs associated with the functionality above, such as a user input confirming connection to a device that has been determined to be in the same medical room and/or an input overriding a determination that a device is not in the same medical room.

Device 300 may include a data source 320 for generating information for transmission over a communication link established by the connection controller 304 and/or a data sink 322 for consuming data received from another device via a communication link established by the connection controller 304. For example, a data source 320 may be an image-generating system that generates images (single snapshot images and/or video frames) that are transmitted to another device via a communication link established by the connection controller 304. A data sink 322 may be a display for displaying images received from an imaging device via a communication link established by the connection controller 304. The one or more displays 316, one or more user input devices 318, data source 320, and/or data sink 322 may be embodied in device 300, as illustrated, or may be embodied in one or more devices that are communicatively connected to device 300 via a wired and/or wireless connection. For example, with reference to FIG. 1, the one or more displays 316, one or more user input devices 318, data source 320, and/or data sink 322 may be embodied in device 112, with the other features of device 300 being embodied in the first device 106.

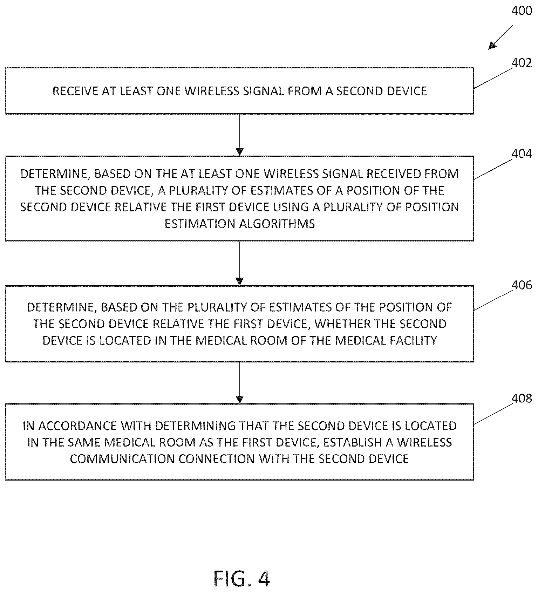

FIG. 4 illustrates an exemplary method 400 for establishing wireless communication between devices in a medical facility, according to the principles described herein. Method 400 can be performed by any of the devices described herein, including the first device 106, the second device 108, and/or the third device 110 of FIG. 1, and/or device 300 of FIG. 3. The steps of method 400 may be performed by one or more processors of a device executing one or more programs stored in a memory of the device.

At step 402, at least one wireless signal is received by a first device from a second device. For example, with reference to FIG. 1, the first device 106 may receive at least one wireless signal from the second device 108. The at least one wireless signal may include a signal that was broadcast by the second device (i.e., a signal that was not specifically intended for the first device). The second device may broadcast the signal so that devices that are within wireless communication range can determine whether they are in the same medical room. The at least one wireless signal may include at least one signal that was transmitted by the second device to the first device in response to a request from the first device. For example, the first device may have transmitted a communication to the second device (or broadcast for any device within wireless communication range) requesting a response that includes information that the first device can use to determine whether the second device (or any other device) is in the same room as the first device. The at least one wireless signal may be a signal associated with a wireless communication protocol, such as ultra-wideband, Wi-Fi, Bluetooth, RFID, ZigBee, or any other wireless communication protocol. The at least one wireless signal may include one or more pulses, including one or more pulses across a wide spectrum of frequencies.

At step 404, the first device determines, based on the at least one wireless signal received from the second device, a plurality of estimates of a position of the second device relative to the first device using a plurality of position estimation algorithms. The plurality of position estimation algorithms may include at least one distance estimation algorithm, such as an algorithm that uses time-of-flight of a signal to determine distance between the transmitter and receiver of a signal. For example, a distance estimation algorithm may be a round-trip time (RTT) measurement, such as implemented by an ultra-wideband communication protocol, that includes measuring the time it takes for a signal to be transmitted from the first device to the second device and for a return signal to be transmitted from the second device to the first device, dividing the time by two, and multiplying by the speed of light. In some examples, a distance estimation algorithm implements phase-based ranging in which time-of-flight is measured using phase data. A distance estimation algorithm may include an algorithm that uses signal strength to determine distance between the transmitter and the receiver of a signal. For example, a received signal strength indicator (RSSI) algorithm may estimate the distance between two devices based on the strength of the received signal, which in general decreases with distance according to the inverse square law and can be affected by environmental factors such as obstacles.

The plurality of position estimation algorithms may include at least one direction estimation algorithm, such as an algorithm that determines the direction from which a signal was received based on the angle of arrival of the signal. In some examples, the first device includes at least one antenna having beam steering capability, and a position estimation algorithm generates an estimate of a direction from which a signal was received based on the direction of beam steering used to receive the signal.

Different position estimates may be determined from the same wireless signal or may be determined from different wireless signals or different sets of wireless signals. For example, a first wireless signal or first set of wireless signals received from the second device may be used to determine a time-of-flight based position estimate and a second wireless signal or second set of wireless signals received from the second device may be used to determine a signal strength-based position estimate. The different wireless signals used for determining different position estimates may correspond to different wireless communication protocols. For example, one or more signals received via an ultra-wideband communication protocol may be used to determine a time-of-flight based position estimate and one or more signals received via a Bluetooth communication protocol may be used to determine a signal strength-based position estimate.

At step 406, the first device determines, based on the plurality of estimates of the position of the second device relative to the first device, whether the second device is located in the same medical room of the medical facility in which the first device is located. Determining whether the second device is located in the same medical room of the medical facility in which the first device is located includes comparing the plurality of estimates of relative position to one another, which may include determining a difference between the estimates of relative position. For example, the first device may compare a relative position estimate that is based on time-of-flight with a relative position estimate that is based on signal strength to determine a difference between the relative position estimates. The difference may be an absolute difference (e.g., two estimates differ by an amount of meters or feet) or may be a relative difference, such as a percentage difference (e.g., estimates differ by ten percent). The first device may determine whether the second device is in the same medical room based on how much the relative position estimates differ. For example, the first device may determine that the second device is in the same medical room when the relative position estimates do not differ or differ by an absolute or relative amount that is less than a threshold. For example, a time-of-flight-based position estimation algorithm and a signal strength-based position estimation algorithm may provide the same or similar position estimates when the one or more signals received from the second device do not pass through any barriers associated with delineating a room, such as walls, windows, doors, floors, or ceilings. For example, where two devices in the same room are separated by 4 meters, a time-of-flight-based position estimate may be 3.85 meters and a signal strength-based position estimate may be 5 meters. The first device may determine that the second device is not in the same medical room when the relative positions differ by an absolute or relative amount that is greater than a threshold. The threshold can be associated with an absolute difference, such as differences between estimates that are one meter or less indicate a device that is in the same medical room, or a relative difference, such as differences that are within ten percent of one another indicate a device that is in the same medical room.

Optionally, the first device takes into consideration whether a given position estimate is greater than (or less than) another position estimate in determining whether the second device is in the same medical room. For example, in general, when the second device is in a different room, a time-of-flight-based position estimate should be significantly less than a signal strength-based position estimate because the signal strength-based position estimation algorithm attributes for signal attenuation (that would be caused by passage of the signal through a room boundary) to distance of travel of the signal. For example, where two devices are located in different rooms and separated by, for example, 4 meters, a time-of-flight-based position estimate may provide a relatively accurate estimate of 3.85 meters, but a signal strength-based position estimate may provide a significantly different estimate of ~7 meters when a solid wall separates the two devices and ~20 meters when a metal wall separates the two devices. Based on this, the first device may check whether the time-of-flight-based position estimate is significantly less than a signal strength-based position estimate and, if not, the first device may determine that there is an error in one or both of the distance estimates. In response to determining that there is an error in one or both of the distance estimates, the first device may return to step 402 to attempt to obtain position estimates that do not have an error. Alternatively, the first device may make a default determination that the second device is, or is not, in the same medical room.

Optionally, determining whether the second device is located in the same medical room includes determining a confidence associated with the likelihood that the second device is in the same medical room or not. The confidence can be determined based on a comparison between the plurality of relative position estimates. For example, the confidence can be determined based on the extent to which relative position estimates differ. For example, the first device may determine a high confidence that the second device is in the same medical room when estimates do not differ or differ by an amount that is far less than a threshold used for determining whether devices are in the same medical room, but may determine a low confidence that the second device is in the same medical room when estimates differ by an amount that is close to the threshold. Similarly, the first device may determine with low confidence that the second device is not in the same medical room based on estimates that differ by an amount that is over a threshold by a relatively small margin and may determine with high confidence that the first device is not in the medical room based on estimates that differ by an amount that is over a threshold by a relatively large margin. A confidence may be determined based on variability over time of relative position estimates. For example, the first device may use a given relative position estimation algorithm multiple times based on multiple signals received at different times and may compare different relative position estimates generated by a given algorithm to assess variability. The first device may determine a low confidence in its determination of whether the second device is in the same medical room when there is high variability across different relative position estimates.

In some examples, a confidence in a determination of whether the second device is in the same medical room with the first device may be determined based on consistency of the determination with a similar determination made by one or more other devices in the medical room that are already communicatively connected to the first device. For example, with reference to FIG. 1, the first device 106 may be communicatively connected to a device 122 that is also in the medical room 102 (e.g., based on the first device 106 previously performing method 400 for device 122), and device 122 may provide an indication of its own determination of whether the second device 108 is in the same medical room (e.g., using method 400) as the first device 106. The first device 106 may determine a relatively high confidence in its determination if its determination matches that of device 122, or a relatively low confidence if not. Optionally, consensus among devices may be used to determine whether a given device is in the same medical room. For example, a given device may be determined to be in the medical room if at least seventy-five percent of the devices in the medical room make a determination based on relative position estimates that the given device is in the same medical room.

At step 408, in accordance with determining at step 406 that the second device is located in the same medical room as the first device, the first device establishes a wireless communication connection with the second device. The first device may establish a wireless communication connection using any suitable wireless connection protocol, such as ultra-wideband, Wi-Fi, Bluetooth, RFID, or ZigBee. Establishing the wireless communication connection with the second device may include exchanging information with the second device that is needed for establishing the wireless communication connection according to the wireless communication protocol. For example, the first device and/or the second device may store passwords or passkeys that may be exchanged for establishing the wireless communication connection.

Once the wireless communication has been established, the first device may wirelessly communicate with the second device in normal fashion according to the wireless communication protocol that is used. For example, the first device may be a control device and may transmit control commands to the second device via the wireless communication connection to control the second device, or the second device may be the control device and may transmit control commands to the first device via the wireless communication connection. Optionally, the first device is a conduit for communication between the second device and another device. For example, with reference to FIG. 1, device 112 may be a controller (e.g., a room controller) that may transmit control commands to the first device 106 for relaying to the second device 108 via the communication connection between the first device 106 and the second device 108, the control commands configured to control one or more functions of the second device 108.

Optionally, prior to establishing the wireless communication connection with the second device, the first device requests user confirmation to establish the wireless communication connection with the second device. For example, with reference to FIG. 2A, the first device may display notification 202 on a display of the first device, or the display communicatively connected to the first device (e.g., a room display, a display of a room controller, a tablet, etc.), indicating to a user that the second device has been determined to be in the same medical room and requesting confirmation that the user would like the first device to connect to the second device.

Whether user confirmation is requested may be based on a risk level associated with at least one of the first device and the second device. As explained above, the risk level can be associated with risk to a patient stemming from devices that are not within the same medical room being incorrectly connected to one another. A high risk level may be associated with control devices that can control other devices that directly interact with a patient such that user confirmation of connection is required before a connection will be made with a control device. For example, a controller that can control an insufflation pump may be associated with a high risk level. A high risk level may be associated with devices that directly interact with a patient, such as the insufflation pump, such that user confirmation is required before a communication will be made with the insufflation pump. Conversely, a low risk level may be associated with devices that typically do not pose risks to a patient, such as display devices or data storage devices.

Whether user confirmation is requested may be based on a confidence in a given determination that the second device is in the same room as the first device. For example, user confirmation may be requested when the second device has been determined to be in the same medical room with low confidence but not with high confidence.

Upon receiving user confirmation to connect to the second device, the first device may establish the wireless communication connection with the second device. If the user declines to connect to the second device, then the first device does not establish a connection with the second device. The first device may add the second device to a list of devices not to connect to such that the first device does not continue to determine whether the second device is in the same medical room. This may avoid a scenario in which a user is repeatedly prompted to confirm whether to connect to the second device. Optionally, the second device may be added to the list of devices not to connect to for a temporary period of time, such as for a number of minutes, hours, or days, such that the first device may again assess whether to connect to the second device once the temporary period of time has elapsed.

Method 400 may include periodically determining whether the second device is still in the same medical room as the first device. Since one or both of the first device and the second device may be mobile devices that may be moved out of the medical room, it may be desirable for a connection between the first device and the second device to be terminated if the devices are no longer in the same medical room. Steps 402 to 406 may be repeated periodically to check whether the second device is in the same medical room.

If a determination is made that the second device is no longer in the same medical room as the first device (because the second device has been moved out of the medical room or because the first device has been moved out of the medical room), a connection between the devices may be broken. Optionally, user confirmation may be requested before breaking the connection. For example, a notification indicating that the first and second devices are no longer in the same medical room may be displayed to a user on a display of the first medical device or a display communicatively connected to the first medical device, and the notification may include a request for confirmation of whether to break the connection. The user confirmation may be requested based on a risk level associated with at least one of the first device and the second device. For example, user confirmation may be requested when the first device or the second device is a device that may be involved directly with patient care, such as a fluid management device, a cauterization device, or a real-time imaging device. User confirmation may not be requested when the first device or the second device is not a device involved directly in patient care, such as a display device or a data storage device.

Optionally, one or more thresholds used for determining whether the second device is no longer in the medical room is different than the corresponding threshold(s) used for determining whether the second device was in the same medical room in the first place. This may increase robustness by lessening false determinations that the second device has moved out of the room.

Method 400 may be repeated for any or all devices that are in wireless communication range of the first device and/or for any or all devices that are in wireless communication range of the first device and that are configured for use in method 400. As such, the first device may establish simultaneous wireless connection with multiple devices. For example, with reference to FIG. 1, the first device 106 may establish a wireless communication connection with the second device 108 and device 122, which is also in the medical room 102. Method 400 may be performed by the second device such that both the first device and the second device both determine whether the other device is in the same medical room. Optionally, a determination by both the first device and the second device that the other device is in the same medical room may be required before a connection is made at step 408.

Optionally, the first device may use position estimates associated with multiple devices for determining a position of the first device in the medical room. For example, the first device may perform method 400 for connecting to the second device and may perform method 400 for connecting to a third device. The first device may use at least one distance estimate relative to the second device determined at step 404 for the first device and at least one distance estimate relative to a third device determined at step 404 for the third device to determine its position within the medical room relative to the second and third devices. The first device may use relative position estimates from any number of devices within a medical room to which it connects to determine its position in the medical room relative to the other devices. In general, the greater the number of devices, the more precise the position estimate may be. Optionally, the first device is provided with the relative positions between other devices in the medical room, which the first device uses to determine its own relative position. For example, with reference to FIG. 1, the first device 106 may receive from one or both of the second device 108 and device 122 a relative position between the second device 108 and device 122, and the first device 106 may use that relative position between the second device 108 and device 122 to determine its own position in the medical room 102 relative to the second device 108 and device 122. As such, the first device may triangulate its position relative to other devices within a medical room based on its relative position to those other devices and the relative positions of those other devices with each other.

Optionally, relative position estimates are exchanged between all wireless devices in a medical room. For example, a table of relative position estimates may be periodically circulated among connected devices in a medical room, and each device may determine its position within the medical room relative to the other devices in the medical room based on the table. One or more of the devices may generate a virtual map of the devices in the medical room based on the table of relative position estimates. The virtual map may provide positions of devices relative to one another. Alternatively, at least one of the devices may have a known fixed position within the medical room, and the virtual map may include the positions of the devices relative to the medical room. For example, the virtual map may include representations of the walls of the room and may include the positions of the devices relative to the walls of the room.

Optionally, the position of a device relative to other devices in a medical room may be used in determining a confidence in a determination that a given device is in the medical room. For example, the first device may be a mobile device that has entered a medical room and may use a determination of its position relative to multiple other devices in the medical room in determining a confidence in its determination that it is in the medical room.

Optionally, at least one device in a medical room that has a known fixed position within the medical room and the known fixed position of the at least one device is used by other devices in the medical room for determining where they are in the medical room. The known fixed position of a device may be broadcast to other devices in the medical room for those devices to determine their position relative to the known fixed position, such as using a suitable triangulation algorithm and/or an algorithm that determines position based on distance between devices and direction of arrival of a signal at a given device. For example, with reference to FIG. 1, medical room 102 may include a plurality of transceivers 124 in known fixed position within the medical room 102 and the first device 106 may determine its position in the medical room 102 based on the known fixed position of the transceivers 124 and the position of the first device 106 relative to the transceivers 124. The position of the first device 106 relative to the transceivers 124 may be determined using only one type of relative position estimate algorithm, such as just time-of-flight or just signal strength. The position of the first device 106 in the medical room 102 may be determined by the first device 106 based on signals received from the transceivers 124 or may be determined by one or more of the transceivers 124 or a device, such as a room controller, communicatively connected to the one or more transceivers 124. For example, with reference to FIG. 1, device 112 may be a room controller that may receive relative position estimates from transceivers 124 (e.g., via communication connection between the first device 106 and the transceivers 124) for the second device 108, and device 112 may determine the position of the second device 108 in the medical room 102 based on the relative position estimates for the second device 108 that are received from the transceivers 124. Alternatively, the first device 106 may determine its position in the medical room 102 based on the known fixed position of just one of the transceivers 124 by determining a distance between the first device 106 and the transceiver 124 and a direction that a signal from the transceiver 124 arrives at the first device 106 (e.g., the first device 106 may include multiple antennas that enable the first device 106 to determine angle of arrival of a signal). As such, the medical room 102 may have just one transceiver 124, and devices may determine their respective positions within the medical room 102 using the known fixed position of the one transceiver 124 based on distance and direction of arrival estimates. Similarly, a single transceiver 124 may determine the position of the first device 106 in the medical room 102 by determining the distance between the single transceiver 124 and the first device and the direction that a signal from the first device 106 arrives at the transceiver 124.

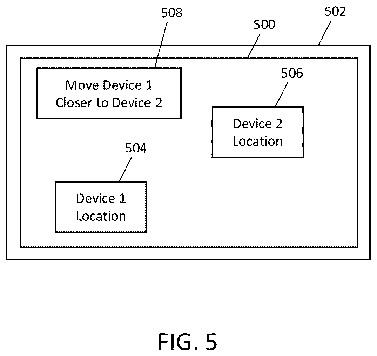

By determining the location of a given device in the medical room, guidance regarding device placement can be provided to a user. FIG. 5 illustrates an exemplary visualization 500 that can be displayed on a display 502 to a user to guide the user in placement of various devices in a medical room. Visualization 500 can be displayed, for example, by a room controller (e.g., device 112 of FIG. 1) of a medical room using one or more displays of the room controller or one or more displays located in the medical room that are communicatively connected to the room controller. Visualization 500 may include a representation of a medical room 504 and one or more representations of medical devices located in the medical room 504, such as device 506 and device 506, positioned in the representation of the medical room 504 according to the positions of the devices 504 and 506 in the medical room 504 determined using transceivers positioned in known fixed positions in the medical room, such as transceiver 124 of FIG. 1. A notification 508 may be provided that guides a user in positioning one or both of devices 504 and 506. In the illustrated example, notification 508 notifies a user that device 504 should be moved closer to device 506. Guidance regarding position of devices with a medical room may be provided based on user (e.g., surgeon) preference, which may help staff set up a medical room in a manner tailored to the preferences of a given user.

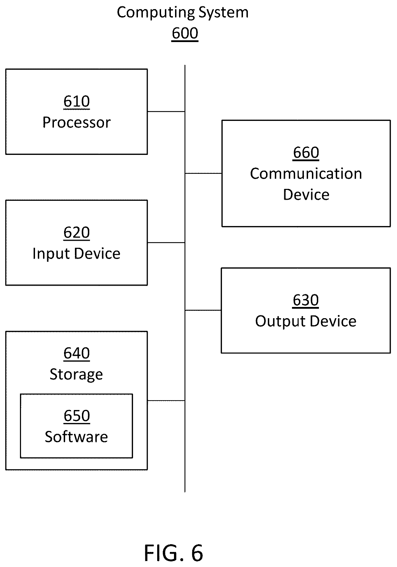

FIG. 6 illustrates an example of a computing system 600 that may be used for any one of the computing systems and devices described herein, such as for any of the devices of FIG. 1 and/or device 300 of FIG. 3. System 600 can be a computer connected to a network. System 600 can be a client computer, a server, a router, a hub, an access point, or any other computing device that can send and/or receive wireless signals or non-wireless signals. As shown in FIG. 6, system 600 can be any suitable type of microprocessor-based system, such as a personal computer, workstation, server, or handheld computing device (portable electronic device) such as a phone or tablet. The system can include, for example, one or more of a processor 610, input device 620, output device 630, storage 640, and communication device 660. Input device 620 and output device 630 can generally correspond to those described above and can either be connectable or integrated with the computer.

Input device 620 can be any suitable device that provides input, such as a touch screen, keyboard or keypad, mouse, gesture recognition component of a virtual/augmented reality system, or voice recognition device. Output device 630 can be or include any suitable device that provides output, such as a touch screen, haptics device, virtual/augmented reality display, or speaker.

Storage 640 can be any suitable device that provides storage, such as an electrical, magnetic, or optical memory, including a RAM, cache, hard drive, removable storage disk, or other non-transitory computer-readable medium. Communication device 660 can include any suitable device capable of transmitting and receiving signals over a network, such as a network interface chip or device. The components of the computer can be connected in any suitable manner, such as via a physical bus or wirelessly.

Software 650, which can be stored in storage 640 and executed by processor 610, can include, for example, the programming that embodies the functionality of the present disclosure (e.g., as embodied in the devices as described above). For example, software 650 can include one or more programs for performing one or more of the steps of method 400.

Software 650 can also be stored and/or transported within any non-transitory computer-readable storage medium for use by or in connection with an instruction execution system, apparatus, or device, such as those described above, that can fetch instructions associated with the software from the instruction execution system, apparatus, or device and execute the instructions. In the context of this disclosure, a computer-readable storage medium can be any medium, such as storage 640, that can contain or store programming for use by or in connection with an instruction execution system, apparatus, or device.

Software 650 can also be propagated within any transport medium for use by or in connection with an instruction execution system, apparatus, or device, such as those described above, that can fetch instructions associated with the software from the instruction execution system, apparatus, or device and execute the instructions. In the context of this disclosure, a transport medium can be any medium that can communicate, propagate, or transport programming for use by or in connection with an instruction execution system, apparatus, or device. The transport-readable medium can include, but is not limited to, an electronic, magnetic, optical, electromagnetic, or infrared wired or wireless propagation medium.

System 600 may be connected to a network, which can be any suitable type of interconnected communication system. The network can implement any suitable communications protocol and can be secured by any suitable security protocol. The network can comprise network links of any suitable arrangement that can implement the transmission and reception of network signals, such as wireless network connections, T1 or T3 lines, cable networks, DSL, or telephone lines.

System 600 can implement any operating system suitable for operating on the network. Software 650 can be written in any suitable programming language, such as C, C++, Java, or Python. In various aspects, application software embodying the functionality of the present disclosure can be deployed in different configurations, such as in a client/server arrangement or through a Web browser as a Web-based application or Web service, for example.

The foregoing description, for the purpose of explanation, has been described with reference to specific aspects. However, the illustrative discussions above are not intended to be exhaustive or to limit the invention to the precise forms disclosed. Many modifications and variations are possible in view of the above teachings. The aspects were chosen and described in order to best explain the principles of the techniques and their practical applications. Others skilled in the art are thereby enabled to best utilize the techniques and various aspects with various modifications as are suited to the particular use contemplated.

In the following, numbered embodiments are described. These numbered embodiments can be considered in the light of the above examples, and summarize particular aspects of the invention.

Embodiment 1: A method for establishing wireless communication between devices in a medical facility, the method comprising, at a first device located in a medical room of the medical facility: receiving at least one wireless signal from a second device; determining, based on the at least one wireless signal received from the second device, a plurality of estimates of a position of the second device relative the first device using a plurality of position estimation algorithms; determining, based on the plurality of estimates of the position of the second device relative the first device, whether the second device is located in the medical room of the medical facility in which the first device is located; and in accordance with determining that the second device is located in the same medical room as the first device, establishing a wireless communication connection with the second device.

Embodiment 2: The method of embodiment 1, wherein the plurality of position estimation algorithms comprises at least one distance estimation algorithm or at least one direction estimation algorithm.

Embodiment 3: The method of embodiment 1 or embodiment 2, wherein the plurality of position estimation algorithms comprises a first distance estimation algorithm that is based on time-of-flight and a second distance estimation algorithm that is based on signal strength.

Embodiment 4: The method of any of the preceding embodiments, wherein determining whether the second device is located in the same medical room as the first device comprises: comparing the plurality of estimates of the position of the second device relative to the first device; and determining a confidence in a determination of whether the second device is located in the same medical room as the first device based on the comparison of the plurality of estimates of the position of the second device relative to the first device.

Embodiment 5: The method of embodiment 4, wherein determining the confidence in a determination of whether the second device is located in the same medical room as the first device comprises evaluating a result of the comparison of the plurality of estimates of the position of the second device relative to the first device based on at least one threshold.

Embodiment 6: The method of embodiment 5, wherein the at least one threshold is associated with wireless signal propagation through at least one type of obstacle.

Embodiment 7: The method of embodiment 6, wherein the at least one type of obstacle comprises a wall, a floor, a door, or a window.

Embodiment 8: The method of any of embodiments 5-7, wherein the at least one threshold is associated with a risk level assigned to at least one of the first device and the second device, the risk level being associated with a risk posed by the at least one of the first device and the second device being communicatively connected to a device in a different medical room.

Embodiment 9: The method of any of embodiments 5-8, wherein the at least one threshold was determined during a set-up of the first device in the medical room.

Embodiment 10: The method of any of embodiments 5-9, wherein the at least one threshold is updated over time based on user input associated with confirmation of whether devices are located in the same medical room.

Embodiment 11: The method of any of embodiments 1-10, comprising, prior to establishing the wireless communication connection with the second device, requesting user confirmation to establish the wireless communication connection with the second device.

Embodiment 12: The method of embodiment 11, wherein the user confirmation is requested based on a risk level associated with at least one of the first device and the second device.

Embodiment 13: The method of any of embodiments 1-12, comprising, after establishing the wireless communication connection with the second device, periodically determining whether the second device is in the same medical room as the first device.

Embodiment 14: The method of embodiment 13, comprising determining that the second device is no longer in the same medical room as the first device and breaking the wireless communication connection with the second device.

Embodiment 15: The method of embodiment 14, comprising, prior to breaking the wireless communication connection with the second device, receiving user confirmation to break the wireless communication connection with the second device.

Embodiment 16: The method of embodiment 15, comprising requesting the user confirmation based on a risk level associated with at least one of the first device and the second device.

Embodiment 17: The method of any of embodiments 1-16, wherein the first device or the second device establishes simultaneous wireless communication connections with a plurality of devices.

Embodiment 18: The method of any of embodiments 1-17, wherein a controller is connected to one of the first device and the second device via a wired connection, the controller configured to send control commands via the wireless communication connection to the other of the first device and the second device.

Embodiment 19: The method of any of embodiments 1-18, comprising, at the first device: receiving at least one wireless signal from a third device; determining, based on the at least one wireless signal received from the third device, a at least one estimate of a position of the third device relative the first device using at least one of the plurality of position estimation algorithms; and determining a position within the medical room of the first device based at least in part on the at least one estimate of the position of the second device relative the first device and at least one of the plurality of estimates of the position of the third device relative the first device.

Embodiment 20: A system comprising a plurality of devices configured for communicating wirelessly, the plurality of devices comprising a first device that is configured to: receive at least one wireless signal from a second device; determine, based on the at least one wireless signal received from the second device, a plurality of estimates of a position of the second device relative the first device using a plurality of position estimation algorithms; determine, based on the plurality of estimates of the position of the second device relative the first device, whether the second device is located in the same medical room of a medical facility as the first device; and in accordance with determining that the second device is located in the same medical room as the first device, establish a wireless communication connection with the second device.

Embodiment 21: The system of embodiment 20, wherein the plurality of position estimation algorithms comprises at least one distance estimation algorithm or at least one direction estimation algorithm.

Embodiment 22: The system of embodiment 20 or embodiment 21, wherein the plurality of position estimation algorithms comprises a first distance estimation algorithm that is based on time-of-flight and a second distance estimation algorithm that is based on signal strength.

Embodiment 23: The system of any of embodiments 20-22, wherein determining whether the second device is located in the same medical room as the first device comprises: comparing the plurality of estimates of the position of the second device relative the first device; and determining a confidence in a determination of whether the second device is located in the same medical room as the first device based on the comparison of the plurality of estimates of the position of the second device relative to the first device.

Embodiment 24: The system of embodiment 23, wherein determining the confidence in a determination of whether the second device is located in the same medical room as the first device comprises evaluating a result of the comparison of the plurality of estimates of the position of the second device relative to the first device based on at least one threshold.

Embodiment 25: The system of embodiment 24, wherein the at least one threshold is associated with wireless signal propagation through at least one type of obstacle.

Embodiment 26: The system of embodiment 25, wherein the at least one type of obstacle comprises a wall, a floor, a door, or a window.

Embodiment 27: The system of any of embodiments 24-26, wherein the at least one threshold is associated with a risk level assigned to at least one of the first device and the second device, the risk level being associated with a risk posed by the at least one of the first device and the second device being communicatively connected to a device in a different medical room.

Embodiment 28: The system of any of embodiments 24-27, wherein the at least one threshold was determined during a set-up of the first device in the medical room.

Embodiment 29: The system of any of embodiments 24-28, wherein the at least one threshold is updated over time based on user input associated with confirmation of whether devices are located in the same medical room.

Embodiment 30: The system of any of embodiments 20-29, wherein the first device is configured to, prior to establishing the wireless communication connection with the second device, request user confirmation to establish the wireless communication connection with the second device.

Embodiment 31: The system of embodiment 30, wherein the first device is configured to request the user confirmation based on a risk level associated with at least one of the first device and the second device.

Embodiment 32: The system of any of embodiments 20-31, wherein the first device is configured to, after establishing the wireless communication connection with the second device, periodically determine whether the second device is in the same medical room as the first device.

Embodiment 33: The system of embodiment 32, wherein the first device is configured to break the wireless communication connection with the second device in response to determining that the second device is not in the same medical room as the first device.

Embodiment 34: The system of embodiment 33, wherein the first device is configured to, prior to breaking the wireless communication connection with the second device, receive user confirmation to break the wireless communication connection with the second device.

Embodiment 35: The system of embodiment 34, wherein the first device is configured to request the user confirmation based on a risk level associated with at least one of the first device and the second device.

Embodiment 36: The system of any of embodiments 20-35, wherein at least one of the first device and the second device is configured to establish simultaneous wireless communication connections with a plurality of devices.

Embodiment 37: The system of any of embodiments 20-36, comprising a controller that is connected to one of the first device and the second device via a wired connection, the controller configured to send control commands via the wireless communication connection to the other of the first device and the second device.

Embodiment 38: The system of any of embodiments 20-37, wherein the first device is configured to: receive at least one wireless signal from a third device; determine, based on the at least one wireless signal received from the third device, a at least one estimate of a position of the third device relative the first device using at least one of the plurality of position estimation algorithms; and determine a position within the medical room of the first device based at least in part on the at least one estimate of the position of the second device relative the first device and at least one of the plurality of estimates of the position of the third device relative the first device.

Embodiment 39. A non-transitory computer-readable medium comprising one or more programs for execution by one or more processors of a first device, the one or more programs including instructions that when executed by the one or more processors cause the first device to: receive at least one wireless signal from a second device; determine, based on the at least one wireless signal received from the second device, a plurality of estimates of a position of the second device relative the first device using a plurality of position estimation algorithms; determine, based on the plurality of estimates of the position of the second device relative the first device, whether the second device is located in the same medical room of a medical facility as the first device; and in accordance with determining that the second device is located in the same medical room as the first device, establish a wireless communication connection with the second device.

Embodiment 40. A computer program product comprising one or more programs for execution by one or more processors of a first device, the one or more programs including instructions that when executed by the one or more processors cause the first device to: receive at least one wireless signal from a second device; determine, based on the at least one wireless signal received from the second device, a plurality of estimates of a position of the second device relative the first device using a plurality of position estimation algorithms; determine, based on the plurality of estimates of the position of the second device relative the first device, whether the second device is located in the same medical room of a medical facility as the first device; and in accordance with determining that the second device is located in the same medical room as the first device, establish a wireless communication connection with the second device.

Although the disclosure and examples have been fully described with reference to the accompanying figures, it is to be noted that various changes and modifications will become apparent to those skilled in the art. Such changes and modifications are to be understood as being included within the scope of the disclosure and examples as defined by the claims. Finally, the entire disclosures of the patents and publications referred to in this application are hereby incorporated herein by reference.

## Claims

1. A method for establishing wireless communication between devices in a medical facility, the method comprising, at a first device located in a medical room of the medical facility:
receiving at least one wireless signal from a second device;
determining, based on the at least one wireless signal received from the second device, a plurality of estimates of a position of the second device relative the first device using a plurality of position estimation algorithms;
determining, based on the plurality of estimates of the position of the second device relative the first device, whether the second device is located in the medical room of the medical facility in which the first device is located; and
in accordance with determining that the second device is located in the same medical room as the first device, establishing a wireless communication connection with the second device.

2. The method of claim 1, wherein the plurality of position estimation algorithms comprises at least one direction estimation algorithm and/or at least one distance estimation algorithm, such as a first distance estimation algorithm that is based on time-of-flight and a second distance estimation algorithm that is based on signal strength.

3. The method of claim 1 or 2, wherein determining whether the second device is located in the same medical room as the first device comprises:
comparing the plurality of estimates of the position of the second device relative to the first device; and
determining a confidence in a determination of whether the second device is located in the same medical room as the first device based on the comparison of the plurality of estimates of the position of the second device relative to the first device.

4. The method of claim 3, wherein determining the confidence in a determination of whether the second device is located in the same medical room as the first device comprises evaluating a result of the comparison of the plurality of estimates of the position of the second device relative to the first device based on at least one threshold.

5. The method of claim 4, wherein the at least one threshold is associated with wireless signal propagation through at least one type of obstacle, such as a wall, a floor, a door, or a window.

6. The method of claim 4 or 5, wherein the at least one threshold is associated with a risk level assigned to at least one of the first device and the second device, the risk level being associated with a risk posed by the at least one of the first device and the second device being communicatively connected to a device in a different medical room.

7. The method of any of claims 4-6, wherein the at least one threshold was determined during a set-up of the first device in the medical room, and/or wherein the at least one threshold is updated over time based on user input associated with confirmation of whether devices are located in the same medical room.

8. The method of any of the preceding claims, comprising, prior to establishing the wireless communication connection with the second device, requesting user confirmation to establish the wireless communication connection with the second device, wherein the user confirmation is for example requested based on a risk level associated with at least one of the first device and the second device.

9. The method of any of the preceding claims, comprising, after establishing the wireless communication connection with the second device, periodically determining whether the second device is in the same medical room as the first device, the method optionally comprising determining that the second device is no longer in the same medical room as the first device and breaking the wireless communication connection with the second device, further comprising, prior to breaking the wireless communication connection with the second device, receiving user confirmation to break the wireless communication connection with the second device, even further optionally comprising requesting the user confirmation based on a risk level associated with at least one of the first device and the second device.

10. The method of any of the preceding claims, wherein the first device or the second device establishes simultaneous wireless communication connections with a plurality of devices.

11. The method of any of the preceding claims, wherein a controller is connected to one of the first device and the second device via a wired connection, the controller configured to send control commands via the wireless communication connection to the other of the first device and the second device.

12. The method of any of the preceding claims, comprising, at the first device:
receiving at least one wireless signal from a third device;
determining, based on the at least one wireless signal received from the third device, a at least one estimate of a position of the third device relative the first device using at least one of the plurality of position estimation algorithms; and
determining a position within the medical room of the first device based at least in part on the at least one estimate of the position of the second device relative the first device and at least one of the plurality of estimates of the position of the third device relative the first device.

13. A system comprising a plurality of devices configured for communicating wirelessly, the plurality of devices comprising a first device that is configured to perform the method of claim 1.

14. The system of claim 13, further configured to perform the method of any of claims 2-12.

15. A computer program product, or a non-transitory computer-readable medium, comprising one or more programs for execution by one or more processors of a first device, the one or more programs including instructions that when executed by the one or more processors cause the first device to:
receive at least one wireless signal from a second device;
determine, based on the at least one wireless signal received from the second device, a plurality of estimates of a position of the second device relative the first device using a plurality of position estimation algorithms;
determine, based on the plurality of estimates of the position of the second device relative the first device, whether the second device is located in the same medical room of a medical facility as the first device; and
in accordance with determining that the second device is located in the same medical room as the first device, establish a wireless communication connection with the second device.
